# EUROPÄISCHE PATENTSCHRIFT

(11) **EP 0 965 645 B1**
(45) Veröffentlichungstag und Bekanntmachung des Hinweises auf die Patenterteilung: **19.07.2006**
(21) Anmeldenummer: 99108754.5
(22) Anmeldetag: 03.05.1999
(51) Int. Cl.: C12P 7/62, C12P 7/64, C07C 69/33, C11D 1/66, C07H 13/02

(54) **Fettsäurepartialester von Polyolen**
Partial polyol fatty acid esters
Esters partiels de polyols et d'acides gras

(30) Priorität: 15.05.1998 DE 19821851
(43) Veröffentlichungstag der Anmeldung: 22.12.1999
(73) Patentinhaber: Goldschmidt GmbH, 45127 Essen (DE)
(72) Erfinder: Grüning, Burghard Dr., 45134 Essen (DE); Hills, Geoffrey, 45255 Essen (DE)

(56) Entgegenhaltungen:
- EP-A- 0 451 461
- WO-A-90/09451
- WO-A-95/14458
- DE-A- 4 407 015
- US-A- 3 173 935
- US-A- 3 211 558
- US-A- 5 247 114
- CHARLEMAGNE D ET AL: "ENZYMATIC SYNTHESIS OF POLYGLYCEROL-FATTY ACID ESTERS IN A SOLVENT-FREE SYSTEM" JOURNAL OF THE AMERICAN OIL CHEMISTS' SOCIETY, AMERICAN OIL CHEMISTS' SOCIETY. CHAMPAIGN, US, Bd. 72, Nr. 1, 1995, Seiten 61-65, XP000490479 ISSN: 0003-021X

## Beschreibung

Gegenstand der Erfindung ist ein Verfahren zur Herstellung von Fettsäurepartialestern von Polyolen oder Polyglycerin, die so erhältlichen Fettsäurepartialester sowie deren Verwendung.

Die DE 38 18 292 A1 betrifft ein Verfahren zur Herstellung von Fettsäure- oder Hydroxyfettsäureestern von Isopropylidenderivaten eines Polyglycerins aus Fettsäurealkylestern, Mono- oder Polyhydroxyfettsäurealkylestern mit C₆-C₂₂ in der Fettsäurekomponente und C₁-C₄ in der Esterkomponente, die im alkalischen Medium mit einem oder mehreren Isopropylidenderivaten eines Polyglycerins umgesetzt werden. Die Umsetzung wird bei Temperaturen von 140 bis 220 °C und im Vakuum bei 5 bis 950 mbar durchgeführt. Der dabei entstehende C₁-C₄ Alkohol wird durch Destillation entfernt und das Reaktionsprodukt gereinigt. Weiterhin wird die Verwendung der so hergestellten Fettsäure- oder Hydroxyfettsäureester von Mono- und/oder Diisopropylidenderivaten des Polyglycerins als Zwischenprodukte für die Herstellung von nichtionogenen Tensiden, als Lösemittel oder Lösungsvermittler sowie für kosmetische Zubereitungen und Hautpflegemittel beschrieben.

Die EP 0 383 405 A1 beschreibt ein Verfahren zur Herstellung von Estern durch Umsetzung einer C₇-C₃₆ Mono- oder Dicarbonsäure und einem C₂-C₈ Monoalkohol in Anwesenheit eines Lipasekatalysators, wobei das Reaktionswasser durch azeotrope Destillation der Mischung aus dem C₂-C₈ Monoalkohol und Wasser entfernt wird. Insbesondere wird die azeotrope Destillation des Alkohols und die Zugabe des Alkohols gleichzeitig in gleichen Raten unter vermindertem Druck durchgeführt. Das Verfahren kann in einem Eintopfsystem bei einer Temperatur unterhalb 80 °C durchgeführt werden. Die Veresterung und die azeotrope Destillation kann ebenso in separaten Reaktionsgefäßen durchgeführt werden, in denen höhere Temperaturen und höhere Drücke angewandt werden können.

In der EP 0 451 461 B1 wird die Verwendung von Mischungen aus Polyglycerinfettsäureestern als Emulgatoren im kosmetischen und pharmazeutischen Zubereitungen beschrieben. Diese sind erhältlich durch partielle Veresterung von Polyglycerinen mit wenigstens einer gesättigten Fettsäure mit 12 bis 22 C-Atomen oder wenigstens einer ungesättigten Fettsäure mit 16 bis 22 C-Atomen, wobei die eingesetzte ungesättigte Fettsäure oder Fettsäuremischung noch bis zu 10 Gew.-% an gesättigten Fettsäuren mit 16 bis 22 C-Atomen enthalten kann. Der Veresterungsgrad der gesättigten oder ungesättigten Fettsäuren in der Mischung liegt zwischen 20 und 70 %.

Die DE 44 07 015 C2 betrifft kosmetische und/oder pharmazeutische Zubereitungen, die dadurch gekennzeichnet sind, daß sie als Emulgatoren Ester der Palmitinsäure mit technischen Triglyceriden mit der Maßgabe enthalten, daß der Gehalt an Monoestern 30 bis 50 Gew.-% beträgt.

In der EP 0 093 602 A2 wird eine Umesterung mittels einem Lipaseenzymsystem als Umesterungskatalysator beschrieben. In einem kontinuierlichen Umesterungsverfahren wird ein Fettsäureesterderivat, insbesondere ein Glycerin, das gegebenenfalls freie Fettsäure enthält, mit einem Enzym als Umesterungskatalysator in Kontakt gebracht, das vorzugsweise 1,3-selektiv und auf einem inerten teilchenförmigen Träger immobilisiert ist. Der Katalysator ist in einem Festbett gepackt und bleibt mit dem Reaktionsgemisch weniger als zwei Stunden in Kontakt. Das Verfahren ist insbesondere geeignet zur Herstellung von POSt- und StOSt-reichen Fetten, die geeignet sind als Kakaobutterersatz (P steht hier für Palmitat, O für Oleat und St für Stearat).

In der WO 90/09451 werden Fettsäureester von Methylglycosiden beschrieben, die durch Umsetzung einer Fettsäure oder eines Fettsäureesters mit einem Methylglycosid in Anwesenheit eines Enzymkatalysators, insbesondere einer Lipase erhalten werden. Die resultierenden Fettsäureester sind vorzugsweise Monoester. Methylglycosidfettsäureester können als oberflächenaktive Mittel in Reinigungsmitteln oder kosmetischen Mitteln eingesetzt werden.

Demgegenüber besteht die Aufgabe der vorliegenden Erfindung insbesondere in der Bereitstellung von Fettsäurepartialestern I von Polyolen oder Polyglycerin mit wenigstens 4 C-Atomen, wenigstens einer primären und wenigstens einer sekundären Alkoholgruppe in den Ausgangsstoffen darin, gegenüber dem Stand der Technik II den Gehalt an Estergruppen der sekundären Alkoholgruppen zu erhöhen. Darüber hinaus besteht die Aufgabe der vorliegenden Erfindung darin, verbesserte Öl-in-Wasser-Emulgatoren zur Verfügung zu stellen, insbesondere Verbindungen mit einem hydrophoben und einem hydrophilen Molekülteil, die üblicherweise HLB-Werte von mehr als 10 aufweisen. Gemäß Römpp Chemie Lexikon, 9. erweiterte Aufl. (1990), S. 1812 - 1913, Stichwort: HLB-System, ist der HLB-Wert ein von Griffin (1950)eingeführtes Maß für die Wasser- bzw. Öl-Löslichkeit von vorwiegend nichtionischen Tensiden und die Stabilität von Emulsionen, zum Beispiel in der Kosmetik. Ein anderes Maß für die Emulsions-Stabilität ist das Zeta-Potential, das im optimalen HLB-Bereich ein Maximum aufweist. Experimentell läßt sich der HLB-Wert zum Beispiel durch die Phenol-Titrationsmethode bestimmen, in dem man die Tensid-Lösung mit 5 %iger Phenollösung bis zur Trübe versetzt. Ferner kann der HLB-Wert (gas-)chromatographisch, durch Bestimmung der Dielektrizitätskonstante oder kolorimetrisch ermittelt werden. Für die Berechnung von HLB-Werten gilt: Für Fettsäureester mehrwertiger Alkohole gilt die Beziehung HLB = 20 (1-VZ/SZ), wobei VZ für die Verseifungszahl und SZ für die Säurezahl des Esters stehen. Für Ethoxylate und deren Ester, bei denen die VZ nur schwer zu bestimmen ist, gilt die Formel HLB = (E + P)/5, wobei E für die Zahl der Ethylenoxideinheiten und P für den Gehalt an mehrwertigen Alkoholen (Angabe in Gew.-%) im Mol. stehen. Es sei darauf hingewiesen, daß diese Berechnungsmethode auf Polypropylenglykolether sowie anionische Tenside nicht angewandt werden kann. Der HLB-Wert eines Tensid- oder Emulgator-Gemisches läßt sich aus den Werten seiner Bestandteile additiv berechnen. Die Skala reicht dabei in der Regel von 1 bis 20, seltener bis 40. Substanzen mit niedrigem HLB-Wert (<10) sind im allgemeinen gute W/O-Emulgatoren, während hydrophilere Tenside mit höherem HLB-Wert als O/W-Emulgatoren wirken.

Im Handel erhältlich sind nichtionische Emulgatoren (Natriumlaurylethersulfat-freie) und Polyethylenglycol-freie Emulgatoren, die vorzugsweise im Kosmetikbereich eingesetzt werden. Mit Hilfe der vorliegenden Erfindung werden entsprechende Polyglycerinester I zur Verfügung gestellt.

Aus der obengenannten DE 38 18 292 A1 sind Triglycerinmonoisostearate bekannt, die die Estergruppe in der mittleren Position tragen. Diese werden durch Schutzgruppen-chemische Reaktion (Isopropylidenderivate) an beiden Enden der Glyceringruppen hergestellt. Hier gegenüber war es eine Aufgabe der vorliegenden Erfindung das Verfahren zur Herstellung der obengenannten Stoffe zu vereinfachen.

Übliche Polyglycerinfettsäureester II, die durch chemische Veresterung hergestellt werden, weisen eine geringe Hydrophilie auf und bilden keine stabilen Öl-in-Wasser-Emulsionen.

Die vorstehend genannten Aufgaben der vorliegenden Erfindung werden in einer ersten Ausführungsform gelöst durch ein Verfahren zur Herstellung von Fettsäurepartialestern I von Polyolen oder Polyglycerin mit wenigstens 4 C-Atomen, wenigstens einer primären und wenigstens einer sekundären Alkoholgruppe der Ausgangspolyole oder Polyglycerin, wobei man in einem ersten Verfahrensschritt die Polyole oder Polyglycerin mit einer Fettsäure oder einem Fettsäurederivat zu einem Fettsäurepartialester II umsetzt und in einem zweiten Verfahrensschritt die erhaltenen Fettsäurepartialester II einer selektiven enzymatischen Spaltung von primären Estergruppen unterwirft.

Mit Hilfe der vorliegenden Erfindung ist es möglich, die Herstellung von Fettsäurepartialestern I von Polyolen oder Polyglycerin mit wenigstens 4 C-Atomen, wenigstens einer primären und wenigstens einer sekundären Alkoholgruppe der Ausgangsstoffe gegenüber dem Stand der Technik deutlich zu vereinfachen. Insbesondere ist eine Schutzgruppenchemie nicht erforderlich. Darüber hinaus ist es möglich, das erfindungsgemäße Verfahren gänzlich ohne Lösungsmittel durchzuführen. Die so erhältlichen Produkte sind besser als Öl-in-Wasser-Emulgatoren geeignet, als Produkte, die ohne die enzymatische Reaktion hergestellt werden. Somit sind beispielsweise Polyglycerinester I mit einem hohen Gehalt an Monoestern (mehr als 50 %) erhältlich, die vorwiegend sekundäre Ester darstellen.

Bedingt durch die Nichtanwendung der Schutzgruppentechnologie werden weniger Nebenprodukte als im Stand der Technik erhalten. Die Produkte sind darüber hinaus reiner als die im Stand der Technik bekannten, da diese keine Katalysator- oder Lösungsmittelreste enthalten. Bedingt durch die regiospezifische Reaktion der Enzyme sind Produkte erhältlich, deren spezifische Zusammensetzungen im Stand der Technik nicht bekannt gewesen sind. Die erfindungsgemäß erhältlichen Fettsäurepartialester I von Polyolen oder Polyglycerin eignen für besondere Anwendungen, beispielsweise als Öl-in-Wasser-Emulgatoren in Emulsionen mit hohem Salzgehalt, als Solubilisierungsmittel oder als oberflächenaktiver Stoff in kosmetischen, pharmazeutischen und reinigenden Zubereitungen.

Besonders bevorzugt wurden Polyole eingesetzt, die wenigstens 4 Hydroxygruppen aufweisen.

In einer weiteren bevorzugten Ausführungsform der vorliegenden Erfindung sind die umzusetzenden Polyole ausgewählt aus Kohlenhydraten, insbesondere Monosacchariden, Oligosacchariden, Polyglycerinen und Alkylglycosiden mit 1 bis 20 C-Atomen im Alkylrest. Die Auswahl der Kohlenhydrate ist dabei nicht eingeschränkt, so daß man vorzugsweise Monosaccharide einsetzt, die ausgewählt sind aus Erythrose, Threose, Arabinose, Ribose, Xylose, Glucose, Mannose, Galactose, Fructose, Sorbose, Sorbit, Mannit und Dulcit. In analoger Weise wie die Monosaccharide sind die Oligosaccharide ausgewählt aus Disacchariden, insbesondere Saccharose, Trehalose, Lactose, Maltose und Cellobiose sowie den Trisacchariden, insbesondere Raffinose. Besonders bevorzugt sind die Zuckeralkohole ausgewählt aus Sorbit, Xylit oder Erythrit, während die Alkylglucoside vorzugsweise Methylglucosid umfassen.

Polyglycerine sind bekanntermaßen Ether von Glycerin, die beispielsweise industriell hergestellt werden durch basenkatalytische Kondensation von Glycerin. Diese Polyglycerine treten auch als Nebenprodukte der Epichlorhydrin-Hydrolyse auf. Die Trennung und Isolation der einzelnen Polyglycerine ist durch Behandlung mit verschiedenen Mitteln möglich. Diglycerin als einfachstes Kondensationsprodukt sowie deren höhere Oligomere sind als synthetische blockbildende Substanzen bekannt, die für eine Reihe von Produkten eingesetzt werden. So sind auch Fettsäureester dieser Polyglycerine prinzipiell im Stand der Technik bekannt. Besonders bevorzugt im Sinne der vorliegenden Erfindung werden technische Gemische von Polyglycerinen eingesetzt, die üblicherweise Diglycerin, Triglycerin, Tetraglycerin und Pentaglycerin enthalten.

Die im Sinne der vorliegenden Erfindung vorzugsweise einzusetzenden Fettsäuren und Fettsäurederivate leiten sich von geradkettigen oder verzweigten, gesättigten, einfach oder mehrfach ungesättigten Fettsäureresten mit 6 bis 24 C-Atomen, insbesondere 12 bis 18 C-Atome auf. Hierbei sind besonders bevorzugt Stearinsäure und Palmitinsäure, die mit Polyglycerinen im Sinne der vorliegenden Erfindung umgesetzt feste Emulgatoren liefern. Wird beispielsweise Isostearinsäure oder Ölsäure als Umsetzungspartner eingesetzt, so werden flüssige Emulgatoren erhalten, insbesondere bei der Umsetzung mit Polyglycerinen.

Als Fettsäurederivate können alle üblichen Derivate eingesetzt werden, die Veresterungsreaktionen eingehen. Dementsprechend sind die Fettsäurederivate besonders bevorzugt ausgewählt aus Fettsäurehalogeniden, Fettsäureanhydriden und Fettsäurealkylestern mit 1 bis 4 C-Atomen im Alkoholrest.

Das Stoffmengenverhältnis von Fettsäure oder Fettsäurederivat zu Polyol oder Polyglycerin kann im Sinne der vorliegenden Erfindung nach allgemein üblichen Verhältnissen eingestellt werden. Besonders bevorzugt im Sinne der vorliegenden Erfindung ist es, das Stoffmengenverhältnis von Fettsäurederivaten zu Polyolen oder Polyglycerin im Verhältnis von 0,1 bis 5:1, insbesondere 0,5 bis 3:1 einzustellen.

Prinzipiell wird die an sich aus dem Stand der Technik bekannte erste Verfahrensstufe dadurch verwirklicht, daß man die Reaktion bei 110 bis 300 °C, insbesondere 240 bis 280°C zur Umsetzung von Polyglycerinen im Verlauf von 2 bis 12 Stunden, insbesondere 3 bis 5 Stunden, insbesondere unter Inertgas, beispielsweise Stickstoff bei destillativer Entfernung des entstehenden Wassers oder Alkohols, gegebenenfalls in Anwesenheit von Lösungsmittel und/oder eines sauren oder basischen Katalysators durchführt. Besonders bevorzugt im Sinne der vorliegenden Erfindung ist es, die Veresterungsreaktion des ersten Verfahrensschrittes in Anwesenheit eines basischen Katalysators, wie beispielsweise einer Fettsäureseife, insbesondere Calciumstearat in Anwesenheit von K₂CO₃ oder Na₂CO₃ durchzuführen. Die Umsetzung von anderen Polyolen, beispielsweise Zuckeralkoholen, erfordert in der Regel niedrige Umsetzungstemperaturen wegen der Instabilität der Alkohole, die bei höheren Temperaturen, oftmals Ringschlußreaktionen erreichen. Beispielsweise wird im Fall der Umsetzung von Sorbit der Temperaturbereich von 110 bis 150 °C bevorzugt.

Die zweite Reaktionsstufe, nämlich die selektive Spaltung der primären Estergruppen erfolgt durch eine enzymatische Reaktion, so daß das erhaltene Produkt vorzugsweise primäre Hydroxylgruppen und sekundäre Fettsäureester sowie eine geringere Zahl von sekundären Hydroxylgruppen enthält. Dies wird erreicht durch Hydrolyse/Alkoholyse der in dem ersten Verfahrensschritt erhaltenen Fettsäurepartialester II durch eine Hydrolyse oder Umesterung gegebenenfalls in üblichen Lösungsmitteln für enzymatische Reaktionen oder Suspendiermedien, die Wasser oder kurzkettige Alkohole, insbesondere mit 1 bis 8 C-Atomen umfassen.

Die enzymatische Spaltung mittels Enzymen, insbesondere immobilisierter Enzyme, wird insbesondere mit solchen Enzymen ausgeführt, die ausgewählt sind aus Lipasen, Esterasen oder Proteasen, insbesondere Lipasen mit definierter Enzymkatalysereaktivität für Esterbindungen, insbesondere Hydrolyse, Synthese und/oder Austausch von Esterbindungen. Derartige Lipasen sind in der eingangs genannten WO 90/09451 beschrieben. Darüber hinaus ist das Produkt Novozym® 435 der Firma Novo Nordisk als immobilisiertes thermostabiles Lipasesystem bekannt und im Handel erhältlich. Dieses Enzym wird besonders bevorzugt im Sinne der vorliegenden Erfindung eingesetzt.

Die Menge des Emzymkatalysators beträgt 0,1 bis 10 Gew-%, vorzugsweise 1 bis 5 Gew.-%. Die Reaktionszeit hängt von der verwendeten Menge und der Aktivität des Enzymkatalysators ab und beträgt beispielsweise bis zu 48 Stunden, vorzugsweise bis zu 24 Stunden.

Das Produktionssystem läßt sich entweder durch einen Rührkesselreaktor oder einen Festbettreaktor charakterisieren.

Im Rührkesselreaktor kann der Enzymkatalysator durch geeignete Maßnahmen wie Filtrieren oder Dekantieren nach beendeter Umsetzung abgetrennt werden und gegebenenfalls mehrmals eingesetzt werden.

Der Festbettreaktor ist mit immobilisierten Enzymen bestückt, wobei die Reaktionsmischung durch die mit Katalysator gefüllte Säule gepumpt wird. Die Reaktionsmischung kann kontinuierlich durch die Säule gepumpt werden, wobei mit der Fließgeschwindigkeit die Verweilzeit und damit der gewünschte Umsatz zu steuern ist. Es ist auch möglich, die Reaktionsmischung im Kreislauf durch die Säule zu pumpen, bis die Umsetzung fertig ist.

Mit einem auf einem Träger immobilisierten Enzym ist es auch möglich, die Reaktion in einem Wirbelbett durchzuführen.

Mit Hilfe des eingangs definierten Verfahrens sind Fettsäurepartialester I von Polyolen oder Polyglycerin erhältlich, die einen gegenüber üblicher Veresterung erhöhten Gehalt an sekundär veresterten Fettsäuren aufweisen. Besonders bemerkbar macht sich dieser Vorteil bei Fettsäurepartialestern I von Polyglycerinen, die mit Hilfe der vorliegenden Erfindung mit einem Gehalt an Monoestern von mehr als 50 bis 90 Gew.-% erhältlich sind.

Besonders bevorzugt im Sinne der vorliegenden Erfindung werden die Fettsäurepartialester I von Polyolen oder Polyglycerin, wie definiert, als Emulgatoren in Öl-in-Wasser-Emulsionen eingesetzt.

Die nachfolgenden Ausführungsbeispiele stellen bevorzugte Umsetzungen der vorliegenden Erfindung dar, sind jedoch nicht geeignet, die Erfindung hierauf zu beschränken.

### Beispiele

### Beispiel 1:

191 g handelsübliches Polyglycerin T (Hersteller Solvay), das wenigstens 40 Gew.-% Tetraglycerin, maximal 50 Gew.-% an Di-, Tri- und Pentaglycerin und maximal 20 Gew.-% Hexa-, Hepta- und Octaglycerin enthält, wurden mit 160 g Ölsäure und 0,7 g Calciumstearat bei 260 °C im Verlauf von 2,5 Std. unter Stickstoffspülung erhitzt, um ein Polyglycerin(mono)oleat II zu bilden. Zu diesem Produkt wurde eine gleiche Menge Isopropanol hinzugegeben und bei 60 °C 1 Gew.-% Enzymkatalysator Novozym® 435 zugegeben. Nach 22 Std. wurde der Katalysator abfiltriert und der verbleibende Isopropanol abdestilliert. Die Zusammensetzungen der Partialester vor II und nach der enzymatischen I Reaktion sind in der Tabelle 1 angegeben. Hierbei handelt es sich um gewichtsbezogene Zusammensetzungen, die mittels Gelpermeationschromatographie erhalten wurden.

### Beispiel 2:

250 g Polyglycerin T wurden unter Zusatz von 207,5 g Stearinsäure bei 260 °C im Verlauf von 4 Std. unter Stickstoffspülung erhitzt. Die erhaltenen Polyglycerinester II wurden mit einer gleichen Menge Isopropanol versetzt, worauf man bei 60°C 1 Gew.-% Enzymkatalysator (Novozym® 435) zugab. Nach 16-stündiger Umsetzung wurde der Katalysator abfiltriert und der Isopropanol abdestilliert. Die Zusammensetzungen der veresterten Produkte vor und nach der enzymatischen Reaktion sind in der Tabelle 1 dargestellt.

### Beispiel 3:

Eine Mischung aus Sorbit (24,2g), Methylpalmitat (108,2 g), Natriumpalmitat (7,35 g) und Na₂CO₃ (0,93 g) wurde gerührt und mit N₂-Einleitung auf 150 °C erhitzt. Nach 12 h wurde die Reaktionsmischung abgekühlt. Die Ausbeute betrug 92 % Sorbitpalmitat II mit der Zusammensetzung der Sorbitester nach GPC-Analyse: Monoester 9 %, Diester 26 %, Triester 36 % und Tetraester +ff 29 %.

10 g dieses Produkts II wurden in 20 g 1-Hexanol bei 60 °C gelöst und 2 g Enzymkatalysator (Novozym® 435) zugegeben. Nach 20 h hatte das Sorbitpalmitat I die Zusammensetzung, die in Tabelle 1 wiedergegeben ist. Da Hexylpalmitat und die Monoester in dem Lösungsmittel die gleichen R_{f}-Werte aufwiesen, konnten diese nicht getrennt werden.

### Tabelle 1:

Zusammensetzung der Polyglycerinester vor II und nach I der enzymatischen Behandlung (Gew.-%)

| Produkt | Monoester | Diester | Triester +ff | Tetraester +ff | Isopropylester |
|---|---|---|---|---|---|
| Beispiel 1 | | | | | |
| Polyglycerin(mono)-oleat II | 40 | 35 | 25 | | - |
| nach der enzymatischen Reaktion I | 32 | 14 | 8 | | 46 |
| neuer Polyglycerinestergehalt | 59 | 26 | 15 | | - |

| Beispiel 2 | | | | | |
|---|---|---|---|---|---|
| Polyglycerin(mono)-stearat II | 39 | 33 | 28 | | - |
| nach der enzymatischen Reaktion I | 36 | 15 | 14 | | 35 |
| neuer Polyglycerinestergehalt | 55 | 23 | 22 | | - |

| Beispiel 3 | | | | | |
|---|---|---|---|---|---|
| Sorbitpalmitatester II | 9 | 26 | 36* | 29 | - |
| nach der enzymatischen Reaktion I | 37** | 16 | 24 | 23 | - |

| | | | | | |
|---|---|---|---|---|---|
| * nur Triester allein | | | | | |
| **Summe aus Monoester und Hexylpalmitat | | | | | |

### Beispiel 4:

Anwendung O/W-Emulgator Polyglycerin T-Stearat aus Beispiel 2, chemisch verestert (PGTS) II und enzymatisch modifiziert I im Vergleich mit über Schutzgruppenchemie synthetisierten Triglycerinmonostearat III wurden jeweils in eine

| | | |
|---|---|---|
| O/W-Emulsion aus | Paraffinöl | 19 % |
| | TEGIN M | 3,0 % |
| | Stearylalkohol | 1,0 % |
| | K-Stearat | 0,03 % |
| + Emulgator | (I) PGTS_{enz} | 2,0 % |
| | (entspricht 1,4 % PG-Ester) | |
| | (II) PGTS | 1,4 % |
| | (III) TGMS | 1,4 % |
| | Wasser | ad 100 |

eingebracht.

Für die Emulsionen I, II und III wurden folgende Eigenschaften bestimmt:

| Emulsion | I | II | III |
|---|---|---|---|
| Dispersionsgrad | feindispers | grobdispers | grobdispers |
| Viskosität (Pas) | 36 | 45 | 8 |
| gemessen bei Raumtemperatur im Brookfield Viskosimeter mit RVT Helipath Spindel bis 104 pm | | | |

| Stabilität bei: | (nach 2 Monaten) | | |
|---|---|---|---|
| Raumtemperatur | stabil | stabil | instabil |
| 40 °C | stabil | stabil | instabil |
| 45 °C | stabil | instabil | instabil |
| Gefrier/Tau-Zyklen | keine Separation | mittlere Separation | starke Separation |
| durch fünfmaliges Abkühlen auf -15 °C und Erwärmen auf Raumtemperatur | | | |

## Patentansprüche

1. Verfahren zur Herstellung von Fettsäurepartialestern (I) von Polyolen oder Polyglycerin mit wenigstens 4 C-Atomen, wenigstens einer primären und wenigstens einer sekundären Alkoholgruppe der Ausgangspolyole oder Polyglycerin, wobei man in einem ersten Verfahrensschritt die Polyole oder Polyglycerin mit einer Fettsäure oder einem Fettsäurederivat zu einem Fettsäurepartialester (II) umsetzt und in einem zweiten Verfahrensschritt die erhaltenen Fettsäurepartialester (II) einer selektiven enzymatischen Spaltung von primären Estergruppen unterwirft.

2. Verfahren nach Anspruch 1, **dadurch gekennzeichnet, daß** man Polyole oder Polyglycerine einsetzt, die wenigstens 4 Hydroxylgruppen aufweisen.

3. Verfahren nach Anspruch 1, **dadurch gekennzeichnet, daß** man Polyole, die ausgewählt sind aus Kohlenhydraten, insbesondere Monosacchariden, Oligosacchariden, Zuckeralkoholen und Alkylglucoside mit 1 bis 20 C-Atomen im Alkylrest, oder Polyglycerine einsetzt.

4. Verfahren nach Anspruch 3, **dadurch gekennzeichnet, daß** man Monosaccharide einsetzt, die ausgewählt sind aus Erythrose, Threose, Arabinose, Ribose, Xylose, Glucose, Mannose, Galactose, Fructose, Sorbose, Sorbit, Mannit, Xylit, Erythrit und Dulcit,
Oligosaccharide einsetzt, die ausgewählt sind aus Disacchariden, insbesondere Saccharose, Trehalose, Lactose, Maltose und Cellobiose und/oder Alkylglucoside einsetzt, die ausgewählt sind aus Methylglucosid und/oder Ethylglucosid.

5. Verfahren nach Anspruch 3, **dadurch gekennzeichnet, daß** man Polyglycerine einsetzt, die Diglycerin, Triglycerin, Tetraglycerin, Pentaglycerin sowie deren Gemische enthalten.

6. Verfahren nach einem oder mehreren der Ansprüche 1 bis 5, **dadurch gekennzeichnet, daß** man Fettsäuren und/oder Fettsäurederivate einsetzt, die geradkettige oder verzweigte, gesättigte, einfach oder mehrfach ungesättigte Fettsäurereste mit 6-24 C-Atomen, insbesondere 12-18 C-Atomen aufweisen.

7. Verfahren nach Anspruch 6, **dadurch gekennzeichnet, daß** man Fettsäurederivate einsetzt, die ausgewählt sind aus Fettsäurehalogeniden, Fettsäureanhydriden und/oder Fettsäurealkylestern mit 1-4 C-Atomen im Alkoholrest.

8. Verfahren nach einem oder mehreren der Ansprüche 1 bis 7, **dadurch gekennzeichnet, daß** man das Stoffmengenverhältnis von Fettsäure und/oder Fettsäurederivat zu Polyol im Verhältnis von 0,1 bis 5:1, insbesondere 0,5 bis 3:1 einstellt.

9. Verfahren nach einem oder mehreren der Ansprüche 1 bis 8, **dadurch gekennzeichnet, daß** man die Reaktion bei 110 bis 300 °C, insbesondere 240 bis 280 °C im Verlauf von 2 bis 12 Std., insbesondere 3 bis 5 Std., zur Umsetzung von Polyglycerinen, insbesondere unter Inertgas, beispielsweise Stickstoff bei destillativer Entfernung des entstehenden Wasser oder Alkohols, gegebenenfalls in Anwesenheit von Lösungsmittel und/oder eines sauren oder basischen Katalysators durchführt.

10. Verfahren nach Anspruch 1, **dadurch gekennzeichnet, daß** man die selektive Spaltung der primären Estergruppen von Fettsäurepartialestern II in einem Lösungsmittel oder Suspendiennedium durchführt, das Wasser oder kurzkettige Alkohole, insbesondere mit 1 bis 8 C-Atomen, Alkane, insbesondere Hexan, Ether, insbesondere Diethylether und/oder Ketone, insbesondere Aceton, umfaßt.

11. Verfahren nach Anspruch 1, **dadurch gekennzeichnet, daß** man die enzymatische Spaltung von Fettsäurepartialestern II mittels Enzymen, Hydrolasen, insbesondere immobilisierten Enzymen durchführt, die ausgewählt sind aus Lipasen, Esterasen oder Proteasen.

## Claims

1. Process for the preparation of fatty acid partial esters (I) of polyols or polyglycerol having at least 4 carbon atoms, at least one primary and at least one secondary alcohol group of the initial polyols or polyglycerol, in a first step reacting the polyols or polyglycerol with a fatty acid or a fatty acid derivative to provide a fatty acid partial ester (II) and in a second step subjecting the fatty acid partial esters (II) obtained to a selective enzymatic cleavage of primary ester groups.

2. Process according to Claim 1, **characterized in that** polyols or polyglycerols are employed which have at least 4 hydroxyl groups.

3. Process according to Claim 1, **characterized in that** polyols selected from carbohydrates, especially monosaccharides, oligosaccharides, sugar alcohols and alkyl glucosides having 1 to 20 carbon atoms in the alkyl radical, or polyglycerols are employed.

4. Process according to Claim 3, **characterized in that** monosaccharides are employed which are selected from erythrose, threose, arabinose, ribose, xylose, glucose, mannose, galactose, fructose, sorbose, sorbitol, mannitol, xylitol, erythritol and dulcitol,
oligosaccharides are employed which are selected from disaccharides, especially sucrose, trehalose, lactose, maltose and cellobiose and/or alkyl glucosides are employed which are selected from methyl glucoside and/or ethyl glucoside.

5. Process according to Claim 3, **characterized in that** polyglycerols are employed which contain diglycerol, triglycerol, tetraglycerol, pentaglycerol and mixtures thereof.

6. Process according to one or more of Claims 1 to 5, **characterized in that** fatty acids and/or fatty acid derivatives are employed which have straight-chain or branched, saturated, mono- or polyunsaturated fatty acid radicals having 6-24 carbon atoms, especially 12-18 carbon atoms.

7. Process according to Claim 6, **characterized in that** fatty acid derivatives are employed which are selected from fatty acid halides, fatty acid anhydrides and/or fatty acid alkyl esters having 1-4 carbon atoms in the alcohol radical.

8. Process according to one or more of Claims 1 to 7, **characterized in that** the amount-of-substance ratio of fatty acid and/or fatty acid derivative to polyol is set at a ratio from 0.1 to 5:1, in particular 0.5 to 3:1.

9. Process according to one or more of Claims 1 to 8, **characterized in that** the reaction is carried out at 110 to 300°C, especially 240 to 280°C, over the course of 2 to 12 hours, especially 3 to 5 hours, for the reaction of polyglycerols, in particular under inert gas, nitrogen for example, with distillative removal of the resulting water or alcohol, in the presence if desired of solvents and/or of an acidic or basic catalyst.

10. Process according to Claim 1, **characterized in that** the selective cleavage of the primary ester groups of fatty acid partial esters II is carried out in a solvent or suspending medium which comprises water or short-chain alcohols, in particular having 1 to 8 carbon atoms, alkanes, especially hexane, ethers, especially diethyl ether, and/or ketones, especially acetone.

11. Process according to Claim 1, **characterized in that** the enzymatic cleavage of fatty acid partial esters II is carried out by means of enzymes, hydrolases, especially immobilized enzymes, which are selected from lipases, esterases or proteases.

## Revendications

1. Procédé pour la préparation d'esters partiels d'acide gras (I) de polyols ou de polyglycérol comprenant au moins 4 atomes de carbone, au moins un groupe alcool primaire et au moins un groupe alcool secondaire des polyols de départ ou du polyglycérol, dans lequel, dans une première étape de procédé, on transforme les polyols ou le polyglycérol avec un acide gras ou un dérivé d'acide gras en un ester partiel d'acide gras (II) et, dans une deuxième étape de procédé, on soumet les esters partiels d'acide gras (II) obtenus à une dissociation enzymatique sélective de groupes esters primaires.

2. Procédé selon la revendication 1, **caractérisé en ce qu'**on utilise des polyols ou des polyglycérols qui présentent au moins 4 groupes hydroxyle.

3. Procédé selon la revendication 1, **caractérisé en ce qu'**on utilise des polyols choisis parmi les hydrates de carbone, en particulier les monosaccharides, les oligosaccharides, les alcools de sucre et les alkylglucosides comprenant 1 à 20 atomes de carbone dans le radical alkyle ou des polyglycérols.

4. Procédé selon la revendication 3, **caractérisé en ce qu'**on utilise
des monosaccharides qui sont choisis parmi l'érythrose, le thréose, l'arabinose, le ribose, le xylose, le glucose, le mannose, le galactose, le fructose, le sorbose, le sorbitol, le mannitol, le xylitol, l'érythritol et le dulcitol,
des oligosaccharides qui sont choisis parmi les disaccharides, en particulier le saccharose, le tréhalose, le lactose, le maltose et le cellobiose et/ou des alkylglucosides qui sont choisis parmi le méthylglucoside et/ou l'éthylglucoside.

5. Procédé selon la revendication 3, **caractérisé en ce qu'**on utilise des polyglycérols, qui contiennent du diglycérol, du triglycérol, du tétraglycérol, du pentaglycérol ainsi que leurs mélanges.

6. Procédé selon l'une ou plusieurs des revendications 1 à 5, **caractérisé en ce qu'**on utilise des acides gras et/ou des dérivés d'acides gras qui présentent des radicaux d'acide gras linéaires ou ramifiés, saturés, monoinsaturés ou polyinsaturés comprenant 6 à 24 atomes de carbone, en particulier 12 à 18 atomes de carbone.

7. Procédé selon la revendication 6, **caractérisé en ce qu'**on utilise des dérivés d'acides gras qui sont choisis parmi les halogénures d'acides gras, les anhydrides d'acides gras et/ou les esters alkyliques d'acides gras comprenant 1 à 4 atomes de carbone dans le radical alcool.

8. Procédé selon l'une ou plusieurs des revendications 1 à 7, **caractérisé en ce qu'**on règle un rapport de quantités d'acide gras et/ou de dérivé d'acide gras à polyol dans un rapport de 0,1 à 5:1, en particulier de 0,5 à 3:1.

9. Procédé selon l'une ou plusieurs des revendications 1 à 8, **caractérisé en ce qu'**on réalise la réaction à 110 jusqu'à 300 °C, en particulier à 240 jusqu'à 280°C en un laps de temps de 2 à 12 heures, en particulier de 3 à 5 heures, pour la transformation de polyglycérols, en particulier sous un gaz inerte, par exemple de l'azote, avec une élimination par distillation de l'eau formée ou de l'alcool formé, le cas échéant en présence de solvant et/ou d'un catalyseur acide ou basique.

10. Procédé selon la revendication 1, **caractérisé en ce qu'**on réalise la dissociation sélective des groupes esters primaires d'esters partiels d'acides gras II dans un solvant ou un agent de mise en suspension, qui comprend de l'eau ou des alcools à courte chaîne, comprenant en particulier 1 à 8 atomes de carbone, des alcanes, en particulier l'hexane, des éthers, en particulier le diéthyléther et/ou des cétones, en particulier l'acétone.

11. Procédé selon la revendication 1, **caractérisé en ce qu'**on réalise la dissociation enzymatique d'esters partiels d'acides gras II au moyen d'enzymes, d'hydrolases, en particulier d'enzymes immobilisées, qui sont choisies parmi les lipases, les estérases ou les protéases.
